Europäisches Patentamt

European Patent Office

Office européen des brevets

(19)

(11) Veröffentlichungsnummer: **0 366 091**
**A2**

(12) **EUROPÄISCHE PATENTANMELDUNG**

(21) Anmeldenummer: 89119757.6

(22) Anmeldetag: 24.10.89

(51) Int. Cl.5: **C12N 9/64 , C12N 15/58 ,**
**A61K 37/547**

(30) Priorität: 24.10.88 DE 3836200

(43) Veröffentlichungstag der Anmeldung:
**02.05.90 Patentblatt 90/18**

(84) Benannte Vertragsstaaten:
**AT BE CH DE ES FR GB GR IT LI LU NL SE**

(71) Anmelder: **BOEHRINGER MANNHEIM GMBH**
**Sandhofer Strasse 112-132**
**D-6800 Mannheim-Waldhof(DE)**

(72) Erfinder: **Stern, Anne, Dr. rer. nat.**
**Karwendelstrasse 10**
**D-8122 Penzberg(DE)**
Erfinder: **Weidle, Ulrich, Dr. rer. nat.**
**Landwehrstrasse 56**
**D-8000 München 2(DE)**
Erfinder: **Mattes, Ralf, Prof. Dr. rer. nat.**
**Friedrich-Zundel-Strasse 14**
**D-7000 Stuttgart 75(DE)**

(74) Vertreter: **Huber, Bernhard, Dipl.-Chem. et al**
**Möhlstrasse 22 Postfach 860 820**
**D-8000 München 86(DE)**

(54) **Domänenduplikations-Muteine des Gewebsplasminogen-Aktivators (t-PA).**

(57) Ein erfindungsgemäßes Gewebsplasminogen-Aktivator (t-PA)-Mutein weist die natürliche Aminosäuresequenz mindestens einer der Domänen Fibrinfinger und Kringel II in verdoppelter Form auf. Zur Herstellung eines solchen t-PA-Muteins insertiert man zwei komplette t-PA-Gene direkt hintereinander in einen geeigneten Vektor, entfernt durch Oligonukleotid gerichtete Mutagenese diejenigen Sequenzen, die für Aminosäuren kodieren, die im Produkt nicht doppelt vorliegen sollen, führt den so erhaltenen mutierten Vektor in eine geeignete Zelle ein und amplifiziert, gewinnt diejenigen Vektoren, in welchen die Deletion stattgefunden hat und exprimiert den Vektor in einer geeigneten Wirtszelle.

EP 0 366 091 A2

## Domänenduplikations-Muteine des Gewebsplasminogen-Aktivators (t-PA)

Die Erfindung betrifft neue t-PA-Muteine, Verfahren zu ihrer Herstellung sowie Mittel zur Gerinnselauflösung, enthaltend diese t-PA-Muteine.

Geronnenes Blut enthält als Hauptkomponente der Proteinmatrix polymeres Fibrin. Dieses Fibrin wird durch Plasmin proteolytisch abgebaut. Plasmin wird aus Plasminogen über Aktivierung durch die sogenannten Plasminogen-Aktivatoren gebildet, z. B. durch den Gewebs-Plasminogen-Aktivator t-PA (tissue-type plasminogen activator). Die enzymatische Aktivität von natürlichem oder aus Säugerzellen gentechnologisch gewonnenem t-PA (katalytische Aktivierung von Plasminogen zu Plasmin) ist in Abwesenheit von Fibrin oder Fibrin-Spaltprodukten (FSP) sehr gering, kann aber in Gegenwart dieser Stimulatoren wesentlich gesteigert werden (um mehr als den Faktor 10).

Der Wirkungsmechanismus von t-PA in vivo ist beispielsweise in Korniger und Collen, Thromb. Hämostasis 46 (1981), 561-565 beschrieben. Die Stimulierbarkeit der Aktivität des t-PA durch Fibrinogen ist ein entscheidender Vorteil gegenüber anderen bekannten Plasminogen-Aktivatoren, wie z. B. Urokinase oder Streptokinase (vgl. z. B. M. Hoylaerts et al., J. Biol. Chem. 257 (1982), 2912-2919; W. Nieuwenhuizen et al., Biochem. Biophys. Acta 755 (1983), 531-533).

In der Klinik hat sich t-PA bereits als wirksames Mittel zur Behandlung von Thromben erwiesen, weshalb versucht wird, es in möglichst großen Mengen herzustellen.

Klinische Untersuchungen haben allerdings gezeigt, daß aufgrund rascher Abbauraten in der Leber große Mengen an t-PA notwendig sind, um in vivo eine effektive Thrombolyse herbeizuführen. Hohe Behandlungsdosen haben jedoch Nebenwirkungen, wie z.B. Blutungen, zur Folge. Es ist deshalb wünschenswert, dieses Thrombolytikum zu verbessern und die Nebenwirkungen wie systemische Fibrinolyse zu reduzieren. Auch eine Reduktion der Behandlungsdosis sollte erreicht werden.

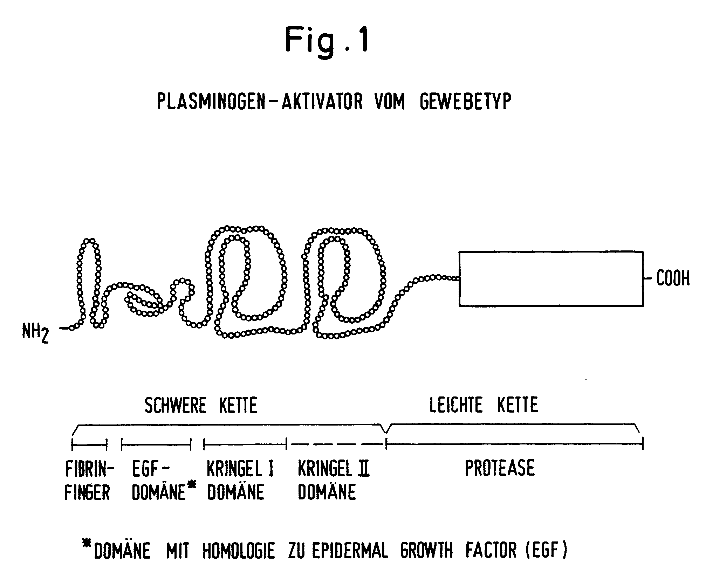

Detaillierte Untersuchungen haben gezeigt, daß t-PA aus Domänen besteht, die in der Fig. 1 gezeigt sind.

In der EP-A 0 241 210 wurden t-PA-Muteine beschrieben, die mehrere Fibrinfingerdomänen enthalten. Hierbei wurde die DNA-Sequenz für die Fibrinfingerdomäne als Restriktionsfragment isoliert und über einen synthetischen Linker vor die erste Aminosäure des Proteins gesetzt. Aufgrund der Verwendung des synthetischen Linkers ergibt sich jedoch eine andere Aminosäureabfolge als der natürlichen Fibrinfingerdomäne entspricht.

Die EP-A-0 231 624 beschreibt ein synthetisch hergestelltes t-PA-Gen, und die daraus hergestellten t-PA-Muteine, welche jede der Domänen höchstens zweimal enthalten. Diese DNA wurde so synthetisiert, daß neue Restriktionsschnittstellen in die Sequenz eingeführt wurden, um das Klonieren der einzelnen Domänen zu erleichtern. Dadurch wurden die Aminosäurepositionen besonders an den Domänenübergängen stark modifiziert. Auch für diese t-PA-Muteine weicht also die Aminosäuresequenz von der Sequenz des natürlichen Proteins ab.

Der Erfindung lag daher die Aufgabe zugrunde, t-PA-Muteine bereitzustellen, die gegenüber dem natürlichen t-PA erhöhte Fibrinbindung und Stimulierbarkeit aufweisen, und nach wie vor alle charakteristischen Eigenschaften des biologischen t-PA's aufweisen.

Diese Aufgabe wird erfindungsgemäß gelöst durch Gewebsplasminogen-Aktivator (t-PA)-Muteine, die die natürliche Aminosäuresequenz mindestens einer der Domänen Fibrinfinger und Kringel II in vervielfachter Form aufweisen.

Unter natürlicher Aminosäuresequenz wird im Rahmen der Erfindung die aus Pennica et al., (1983) Nature 301, 214 bekannte Aminosäuresequenz für die einzelnen Domänen verstanden. Die Vervielfachung erfolgt gemäß den Exon-Intron-Grenzen der natürlichen t-PA-DNA-Sequenz. Es ergibt sich folglich ein Protein, das zwar mindestens eine der beiden Domänen in mehrfacher Form enthält, jedoch keine zusätzliche fremde Aminosäure in seiner Sequenz aufweist, und auch keine Deletion einer Aminosäure. Vorzugsweise liegen die vervielfachten Sequenzen doppelt vor, sie können jedoch auch drei- oder vierfach oder noch öfter vorliegen.

In einer bevorzugten Ausführungsform der Erfindung ist bei dem t-PA-Mutein zusätzlich die Aminosäuresequenz der Domäne EGF oder/und Kringel I deletiert. Durch die Deletion der EGF-Domäne ergibt sich eine wesentlich verlängerte Halbwertszeit gegenüber natürlichem t-PA, da diese Domäne für den raschen Abbau des Proteins in der Leber nötig ist. Die Kringel I-Domäne kann ohne Verlust von Aktivität deletiert werden, es ergibt sich dabei der Vorteil, daß die Aminosäuresequenz durch die Verdopplung nicht übermäßig verlängert wird.

In einer bevorzugten Ausführungsform der Erfindung liegt im t-PA-Mutein die Fibrinfingerdomäne, nämlich die Aminosäuren 4 bis 49 verdoppelt vor. In einer besonders bevorzugten Ausführungsform der Erfindung sind zusätzlich die Aminosäuren der EGF-Domäne, nämlich die Aminosäuren 50 bis 87 und die Aminosäuren der Kringel I-Domäne, Aminosäuren 88 bis 175 deletiert.

In einer anderen bevorzugten Ausführungsform der Erfindung ist die Aminosäuresequenz der Kringel II-Domäne, Aminosäure 176 bis 261 verdoppelt. In wiederum einer besonders bevorzugten Ausführungsform sind zusätzlich die Aminosäuren der EGF-Domäne und der Kringel I-Domäne deletiert.

In einer dritten bevorzugten Ausführungsform sind die EGF- und die Kringel I-Domäne deletiert und die Fibrinfinger- und die Kringel II-Domäne verdoppelt.

Je nachdem, ob das erfindungsgemäße t-PA-Mutein in Prokaryonten oder Eukaryonten exprimiert wurde, liegt es glykosyliert oder unglykosyliert vor. In einer bevorzugten Ausführungsform der Erfindung ist das t-PA-Mutein glykosyliert, und gleicht daher besonders stark dem natürlichen t-PA.

Ein weiterer Gegenstand der Erfindung ist ein Verfahren zur Herstellung eines erfindungsgemäßen t-PA-Muteins, wobei man mehrere komplette t-PA-Gene in Tandemanordnung in einen geeigneten Vektor insertiert, durch Oligonukleotid-gerichtete Mutagenese die Sequenzen entfernt, die für Aminosäuren derjenigen Proteindomänen kodieren, die im Produkt nicht mehrfach vorliegen sollen, den so erhaltenen mutierten Vektor in eine geeignete Zelle einführt und amplifiziert, diejenigen Vektoren gewinnt, in welchen die Deletion stattgefunden hat und den Vektor in einer geeigneten Wirtszelle exprimiert. Die Oligonukleotid-gerichtete Mutagenese wird hierbei nach an sich bekannten Methoden durchgeführt (z.B. Morinaga et al., (1984) Biotechnology 2, 634).

In einer bevorzugten Ausführungsform der Erfindung entfernt man vor der Insertion der beiden t-PA-Gene in einen Vektor daraus ebenfalls durch Oligonukleotid-gerichtete Mutagenese die EGF-Domäne und/oder Kringel I-Domäne.

Zur Oligonukleotid-gerichteten Mutagenese werden Oligonukleotide verwendet, die zu Nukleotiden der 3'- bzw. 5'-Sequenz der zu verbindenden DNA-Sequenzen komplementär sind. Bevorzugt verwendet man ein Oligonukleotid mit 18 bis 26 Nukleotiden Länge.

In einer weiteren bevorzugten Ausführungsform der Erfindung werden zuerst die EGF- und Kringel I-Domäne entfernt, und dann die DNA-Sequenz für die Domänen Fibrinfinger (F), Kringel II (K2) und die leichte Kette (Protease) = (L) zweimal hintereinander in einen Vektor eingebracht, wodurch sich die Sequenz FK2LFK2L ergibt, und daraus durch Oligonukleotid-gerichtete Mutagenese die Sequenzen zwischen den beiden F-Domänen, also K2 und L, oder die Sequenzen zwischen den beiden K2-Domänen, also L und F entfernt. Es ergeben sich daraus Vektoren, die die DNA-Sequenz für ein Mutein mit den Domänen FFK2L oder FK2K2L enthalten.

In einer bevorzugten Ausführungsform der Erfindung verwendet man als t-PA-DNA die t-PA-cDNA. Die erhaltenen Vektoren werden bevorzugt in Prokaryontenzellen amplifiziert, wobei bevorzugt ein E. coli Stamm verwendet wird, der ein lacI$^q$-Plasmid enthält. Nach Selektion der Vektoren, in denen die Deletionen stattgefunden haben, wird in einer bevorzugten Ausführungsform der Erfindung der Vektor in Eukaryonten exprimiert. Die Expression ist zwar auch in Prokaryonten möglich, hieraus ergibt sich jedoch ein nicht-glykosyliertes Produkt. Da ein glykosyliertes Produkt jedoch dem natürlichen t-PA entspricht, wird die Expression in Eukaryonten bevorzugt. In einer besonders bevorzugten Ausführungsform wird die Expression in CHO-Zellen durchgeführt. Bevorzugt wird zur Expression in Eukaryonten als Vektor ein auf Dihydrofolatreduktase oder auf Bovine-papilloma-Virus basierender, amplifizierbarer Vektor verwendet.

In besonders bevorzugten Ausführungsformen werden die Plasmide pA18/1, pA19/1 in E. coli exprimiert. Ein weiterer Gegenstand der Erfindung sind die Plasmide pA18/1, pA19/1, die die Mutein-DNA für die Domänen FFK2L bzw. FK2K2L enthalten, die Plasmide pA24 und pA25, welche die DNA-Sequenz für dieselben Domänen enthalten, jedoch zusätzlich die eukaryontische Signalsequenz enthalten und die Plasmide pSV-FFK2L-dhfr und pSV-FK2K2L-dhfr, welche amplifizierbare eukaryontische Expressionsvektoren für FFK2L und FK2K2L darstellen.

Ein weiterer Gegenstand der Erfindung ist ein Mittel zur Gerinnselauflösung, enthaltend ein erfindungsgemäßes Gewebsplasminogen-Aktivator(t-PA)-Mutein, das mindestens die Domänen Fibrinfinger und/oder Kringel II vervielfacht enthält. Besonders bevorzugt enthält das Mittel ein t-PA-Derivat, bei dem zusätzlich die EGF-und die Kringel I-Domäne deletiert sind. In einer besonders bevorzugten Ausführungsform enthält das Mittel das Mutein mit den Domänen FFK2L oder FK2K2L.

Die Erfindung schafft neue pharmakologisch wirksame t-PA-Muteine, die erhöhte Fibrinbindung und Fibrinstimulierbarkeit aufweisen, und in bevorzugten Ausführungsformen eine deutlich erniedrigte Abbaurate in der Leber aufweisen. Zusätzlich ergibt sich bei den t-PA-Muteinen eine erhöhte Produktionsrate in Eukaryonten.

Es wird daher ermöglicht, Arzneimittel zur Gerinnselauflösung bereitzustellen, bei denen ein wesentlich geringerer Gehalt an t-PA eine deutlich erhöhte Wirksamkeit zeigt.

Die folgenden Beispiele erläutern in Verbindung mit den Abbildungen die Erfindung weiter.

Fig. 1 zeigt die Anordnung der Domänen von t-PA.

Fig. 2 zeigt schematisch die Herstellung der Plasmide pA18/1 und pA19/1.

Fig. 3 zeigt schematisch die Herstellung der Plasmide pSV-FFK2L-dhfr und pSV-FK2K2L-dhfr.

**Beispiel 1**

Herstellung von FFK2L

a) Vorkonstruktion FK2L/FK2L

Das Ausgangsplasmid pREM 7685, beschrieben in EP-A 0 242 836, enthält folgende Komponenten: tac-Promotor, lac-Operator-Region mit einem ATG-Startcodon, die kodierende Region für das Mutein FK2L, den Transkriptionsterminator aus pKK223-3, ein ß-Lactamase-Gen, ein Kanamycin-Resistenz-Gen und den Replikationsursprung (Origin) des Plasmides pACYC177. Die Sequenz des Muteins FK2L setzt sich zusammen aus den Nukleotiden 190-336 (F-Domäne), 715-1809 (K2-Domäne, Protease, geringer Anteil 3' UT (nicht translatierte 3'-Region)) und einem ATG-Startcodon. Die Nukleotidpositionen werden gemäß der von Pennica et al. (Nature 301 (1983), 214) beschriebenen Sequenz angegeben.

Zur Duplikation der t-PA-Mutein-Expressionskassette, bestehend aus Promotor/Operator-Region und dem für das Mutein kodierenden Bereiches einschließlich der Transkriptionssignale, wurde das Plasmid pREM 7685 mit dem Restriktionsenzym XhoII gespalten. Der Restriktionsansatz wurde im Agarose-Gel elektrophoretisch aufgetrennt und ein etwa 1480 bp großes Fragment isoliert.

In einem zweiten Ansatz wurde pREM 7685 mit dem Enzym BamHI linearisiert und anschließend mit alkalischer Phosphatase aus Kälberdarm behandelt.

Nach Ligierung der beiden gewonnenen DNA-Fragmente wurde der Ligierungs-Ansatz in lacI$^q$-Plasmid enthaltende E. coli-Zellen, DSM 3689, transformiert. Plasmid-tragende Transformanten werden durch Zugabe von 25 mg/ml Kanamycin zum Nährmedium selektioniert.

Durch Restriktionsenzym-Analyse konnten unter den Transformanten jene Klone ausgewählt werden, die das Plasmid pA15 enthielten. Dieses Plasmid unterscheidet sich vom Ausgangsplasmid pREM 7685 u.a. durch das Auftreten eines etwa 1480 bp großen zusätzlichen Smal-Fragments, das sowohl den Einbau als auch die gewünschte Orientierung des XhoII-Fragmentes bestätigt. pA15 enthält somit die obengenannte Expressionskassette zweifach und zwar in tandem-Anordnung.

b) Mutagenese

Als Ausgangsplasmid zur Konstruktion des Mutein-Gens FFK2L diente pA15. Zur Deletion der gewünschten Bereiche aus pA15 wurde die Methode von Morinaga et al. (1984) Biotechnology 2: 634 angewendet. Zur Heteroduplexbildung wurden aus pA15 zwei Fragmente isoliert. Fragment A: pA15 wurde mit den beiden Restriktionsenzymen BamHI und Sall (polylinker Region) gespalten. Die Spaltprodukte wurden gelelektrophoretisch aufgetrennt und das große BamHI/Sall-Fragment aus dem Gel eluiert. Fragment B: pA15 wurde mit dem Restriktionsenzym XhoI linearisiert. Das linearisierte Plasmid wurde ebenfalls durch Gelelektrophorese präparativ erhalten. Für die Mutagenese wird folgendes Oligonukleotid synthetisch hergestellt:
5' GCCTGTCAAAGTGATCTGCA 3'.

Zur Heteroduplex-Bildung wurden Fragment A, Fragment B (jeweils 450 fmol) und das Oligonukleotid (75 pMol) gemischt und in Gegenwart von 50 mM NaCl, 10 mM Tris HCl, pH 7,5 und 10 mM MgSO$_4$ zunächst drei Minuten bei 100°C inkubiert und sofort auf Eis überführt. Die Renaturierung der DNS erfolgte für 30 Minuten bei 60°C. Zur Reparatursynthese wurde der Reaktions-Ansatz eingestellt auf: Desoxynukleotidtriphosphate (0,25 mM), ATP (1 mM), NaCl (100 mM), Tris-HCl pH 7,5 (6,5 mM), MgCl$_2$ (8 mM), ß-Mercaptoethanol (1 mM), Klenow-Fragment der DNA-Polymerase aus E. coli (0,125 U/µl Ansatz) und T4-Ligase (0,1 U/µl Ansatz). Die Reparatursynthese erfolgte für 4 Stunden bei 16°C. Anschließend wurde dieser Ansatz in E. coli-Zellen mit einem lacI$^q$-Plasmid, DSM 3689, transformiert und die Transformanten durch Zugabe von 25 µg/ml Kanamycin zum Nährmedium selektioniert.

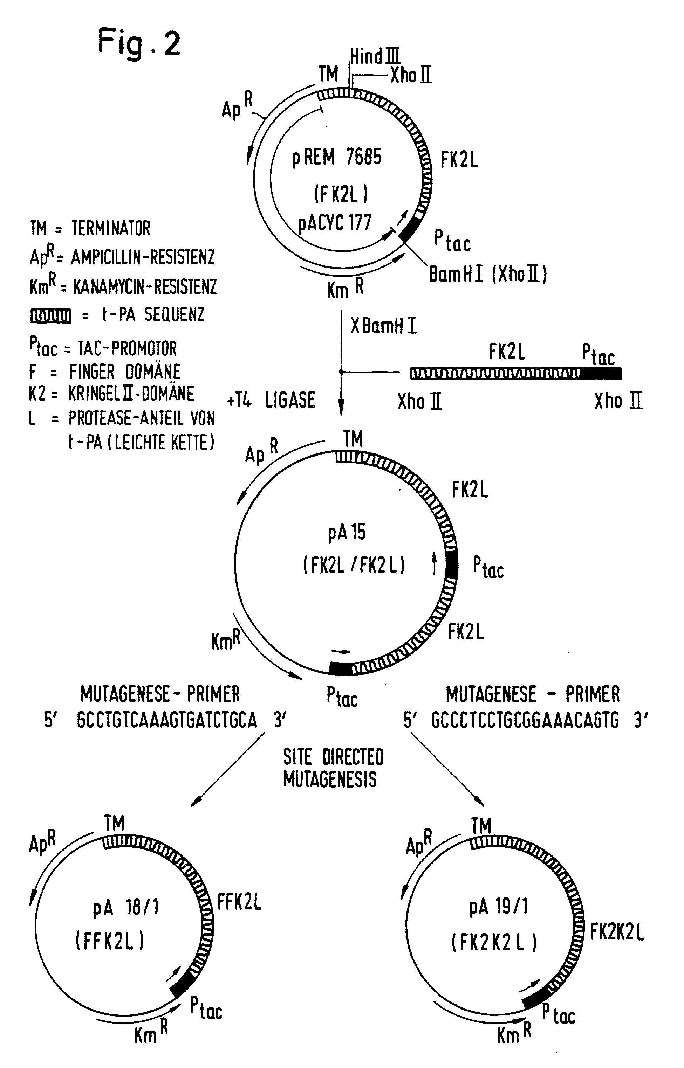

Mit Hilfe der Koloniehybridisierungs-Technik unter Verwendung des oben beschriebenen Mutagenese-Oligonukleotids als Sonde konnten jene Klone ausgewählt werden, die das Plasmid pA18/1 tragen. Dieses Plasmid unterscheidet sich vom Ausgangsplasmid pA15 unter anderem durch das Fehlen einer Smal-Schnittstelle, die zunächst durch die Duplikation der Mutein-Expressionskassette eingeführt worden war. Figur 2 zeigt schematisch die Herstellung von pA18/1.

c) Expression von FFK2L in E. coli

Das Plasmid pA18/1 wurde in E. coli-Zellen, DSM 3689, transformiert, die ein lacI$^q$-Plasmid enthalten. Die Plasmid-tragenden Zellen wurden im Nährmedium bis OD$_{550}$ = 0,4 unter Belüftung bei 37°C kultiviert und durch Zusatz von 0,2 % Lactose oder 5 mM IPTG (Isopropyl-1-thio-ß-D-galactopyranosid) für drei Stunden induziert. Nach dem in DE-A 35 37 708 beschriebenen Protokoll wurden die sogenannten Inclusionbodies vom Zellprotein abgetrennt und das mutierte t-PA-Protein renaturiert. Das so erhaltene Protein wies ein Molekulargewicht von ca. 49 kD auf und bindet an polyklonale Antikörper gegen menschliches t-PA (hergestellt in Ziege). Das renaturierte Mutein (Renaturierung wie in EP-A 0 219 874 beschrieben) zeigte sowohl amidolytische Aktivität als auch Fibrinstimulierbarkeit im plasminogenolytischen Test.

d) Konstruktion von FFK2L mit eukaryontischer Signal-Sequenz

Um das Mutein-Gen mit der Leadersequenz und der 3' UT (3' untranslatierte Region), entsprechend der originalen cDNA-Sequenz, zu versehen, wurden folgende Fragmente isoliert: Aus Plasmid p7.1, DSM 4719, das im Polylinker von pUC12 die 5'UT (5' untranslatierte Region), die Leadersequenz und die N-terminale Sequenz von t-PA bis einschließlich Nukleotidposition 208 enthält, wurde ein ca. 2,7 kb großes PstI/HindIII-Fragment erhalten. Aus dem Plasmid pePA98.1 (siehe EP-A 0 242 835) wurde ein HindIII/ScaI-Fragment isoliert, das etwa 1200 bp umfaßt und die kodierende Region für die Protease und die 3'UT enthält. Aus pA18/1 wurde zunächst ein BamHI/ScaI-Fragment präpariert, aus dem durch partielle Restriktion mit PstI ein etwa 470 bp umfassendes PstI/ScaI-Fragment gewonnen wurde. Dieses Fragment enthält die kodierende Region für die duplizierte Domäne F und die K2-Domäne. Die Fragmente wurden ligiert und in E. coli mit lacI^q-Plasmid, DSM 3689, transformiert. Kanamycin resistente Transformanten wurden mit dem obengenannten Mutageneseprimer gescreent. Das richtige Plasmid, pA25, ergibt bei Restriktion mit PstI Fragmente der Längen 2700 bp (pUC 12 Vektor); 790 bp, 730 bp, 140 bp und 70 bp (t-PA-Fragmente).

## Beispiel 2

Herstellung von FK2K2L

a) Mutagenese und Expression in E. coli

Aus dem FK2L-Doppelgen-Plasmid pA15 wurden gemäß Beispiel 1 die beiden Fragmente A und B isoliert. Zur Heteroduplexbildung wurde in diesem Fall jedoch folgendes Oligonukleotid eingesetzt:
5' GCCCTCCTGCGGAAACAGTG 3'.
Alle experimentellen Schritte wurden, wie bereits im Beispiel 1 beschrieben, durchgeführt. Durch Koloniehybridisierung mit dem obengenannten Mutagenese-Oligonukleotid wurden diejenigen Klone ermittelt, die das gesuchte Plasmid pA19/1 (5,2 kb) enthalten. Dieses Plasmid wurde durch Restriktions-Analyse charakterisiert und auf das Fehlen einer SmaI-Schnittstelle überprüft.

Figur 2 zeigt schematisch unter anderem die Herstellung von pA19/1. pA19/1 wurde in E. coli, DSM 3689, transfektiert und das Mutein FK2K2L exprimiert. Das Protein hat eine Größe von etwa 52000 Dalton. Das Mutein bindet an polyklonale humane t-PA-Antikörper (hergestellt in Ziege) und weist amidolytische Aktivität und Fibrinstimulierbarkeit im plasminogenolytischen Test auf.

b) Rekonstitution von FK2K2L mit eukaryontischer Signal-Sequenz

Zum Anfügen der originalen Leadersequenz an das Mutein-Gen FK2K2L wurden folgende Fragmente isoliert und ligiert: ein 2,7 kb großes BamHI/HindIII-Fragment aus p 7.1, DSM 4719, ein etwa 0,2 kb großes BamHI/SspI aus pA20 (in die PstI-Schnittstelle von p 7.1 wurde ein 450 bp PstI-Fragment aus der t-PA kodierenden Sequenz eingefügt) und ein etwa 1,3 kb großes SspI/HindIII-Fragment aus pA19. Der Ligierungs-Aansatz wurde in E. coli-Zellen, DSM 3689, transformiert und die Plasmid-enthaltenden Transformanten durch Zugabe von 50 μg/ml Ampicillin zum Nährmedium selektioniert. Das gewünschte Plasmid pA24 wurde durch detaillierte Restriktionsenzymanalyse charakterisiert.

## Beispiel 3

Konstruktion amplifizierbarer eukaryotischer Expressionsvektoren für FFK2L und FK2K2L

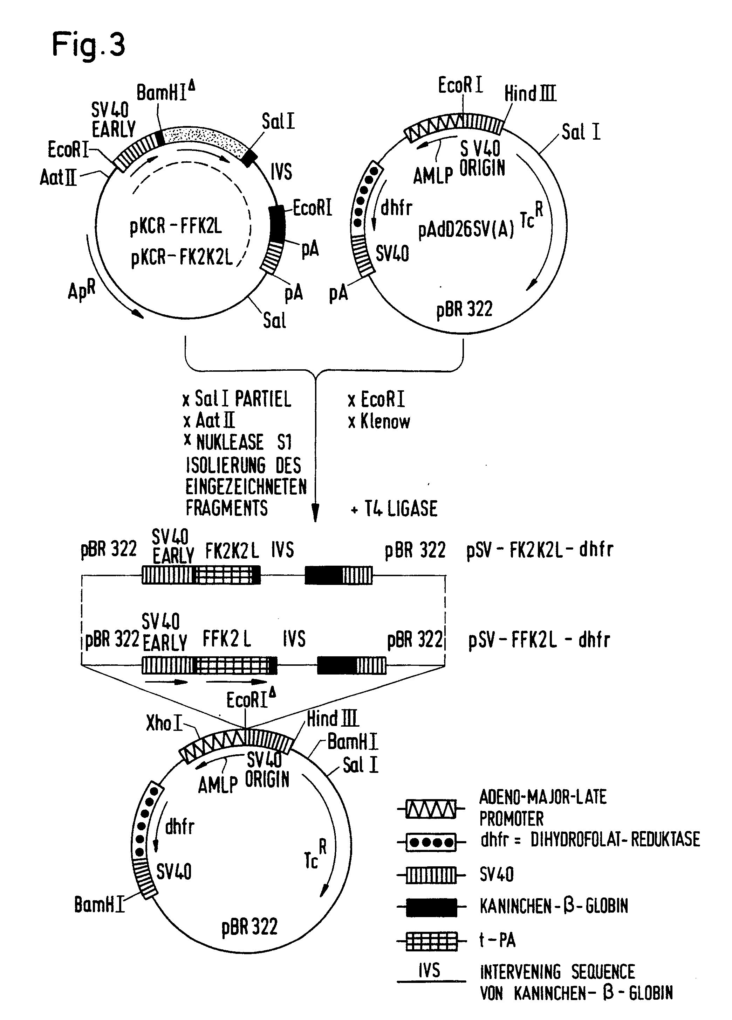

FFK2L und FK2K2L cDNA wurden als Xba-HindIII-Fragmente aus pA25 und pA24 isoliert und in die BamHI-Stelle des eukaryontischen Expressions-Vektors pKCR (O'Hare et al., Proc. Natl. Acad. Sci. USA 78 (1981) 1527-1531) einligiert, nachdem die Enden sowohl von FFK2L und FK2K2L c-DNA und des Vektors mit Nuklease S1 glattgemacht (bluntiert) worden waren. Der Ligierungs-Ansatz wurde in E. coli, HB101 (DSM 1607) transfiziert, Plasmid-tragende Kolonien auf Nährmedium mit 50 μg/ml Ampicillin isoliert und Plasmide mit der richtigen Orientierung (syn) von FFK2L und FK2K2L bezüglich des frühen Promotors von SV40 durch Restriktionskartierung bestimmt. Die Expressions-Vektoren werden als pKCR-FFK2L und pKCR-FK2K2L bezeichnet. Aus diesen Plasmiden wurde die jeweilige Expressionskassette als AatII-SalI-Fragment isoliert. Dazu wurden pKCR-FFK2L und pKCR-FK2K2L partiell mit SalI und anschließend mit AatII gespalten, die Enden mit Nuklease S1 glattgemacht und die entsprechenden Fragmente (in der Abbildung mit einer unterbrochenen Linie dargestellt) nach Auftrennung auf einem niedrigschmelzenden Agarose-Gel isoliert. Diese Fragmente wurden in die mit Klenow aufgefüllte EcoRI-Stelle des amplifizierbaren Vektors pAdD26SV(A) (Kaufman und Sharp, 1982, J. Mol. Biol. 159, 601-621) einligiert. Die ligierten Reaktions-Mischungen wurden in E. coli HB 101 transfiziert und Kolonien auf Tetracyclin-haltigem Nährmedium (10 μg/ml) isoliert. Die entsprechenden Rekombinanten wurden durch Restriktions-Analyse ermittelt, welche auch die Orientierung der Expressionskassetten für die t-PA-Derivate relativ zum Expressionsvektor festlegte. Die isolierten Expressionsvektoren wurden als pSV-FFK2L-dhfr und pSV-FK2K2L-dhfr bezeichnet (Fig. 3).

pSV-FFK2L-dhfr ist dadurch charakterisiert, daß bei Restriktion mit SalI zwei Fragmente der Längen 2470 bp und 7100 bp erhalten werden.

pSV-FK2K2L-dhfr ist dadurch charakterisiert, daß bei Restriktion mit SalI zwei Fragmente der Längen 2470 bp und 6860 bp erhalten werden.

**Beispiel 4**

Etablierung von CHO-Zellen, welche konstitutiv FFK2L und FK2K2L sezernieren.

CHO dhfr⁻-Zellen (DUKX, DHFR⁻), ECACC 88072103, wurden wie von Urlaub und Chasin beschrieben, propagiert (Proc. Natl. Acad. Sci. USA 77 (1980), 4216- 4220). Die Zellen werden in alpha-Medium propagiert, welches mit Adenosin, Deoxyadenosin und Thymidin und 10%igem fötalem Kälberserum mit Penicillin und Streptomycin supplementiert ist. Zur permanenten Expression wurden Calciumphosphat-Präzipitate mit 20 μg der geeigneten Expressionskonstrukte, wie beschrieben (Graham und Van der Eb, Virology 52 (1973), 456-467) in einem Endvolumen von 4 ml hergestellt. 1 ml des Präzipitats wurde zu 3 x 10⁵ bis 1 x 10⁶ Zellen in 10 ml Medium in 9 cm Kulturschalen zugegeben. Die Zellen wurden 6 bis 18 Stunden mit dem Präzipitat inkubiert, das Medium entfernt und mit 10 ml TBS (25 mmol/l, Tris-HCl, pH 7,4, 137 mmol/l NaCl, 5 mmol/l KCl, 0,6 mmol/l Na₂HPO₄, pH 7,4) gewaschen, mit 2 ml 20%igem Glycerin in Trisbuffered saline (TBS) 3 bis 3,5 Minuten inkubiert, mit TBS gewaschen und dann im geeigneten Medium inkubiert. Die CHO-Zellen wurden 48 Stunden nach Transfektion 1:10 umgesetzt und in selektivem Medium (Kaufman und Sharp, J. Mol. Biol. 159 (1982) 601-621) vermehrt. Das Selektionsmedium ist alpha-Medium mit dialysiertem 10%igen fötalem Kälberserum, enthält also keinen Zusatz an Adenosin, Deoxyadenosin und Thymidin. Klone wurden 2 bis 3 Wochen nach Transfektion nach Trypsinieren isoliert, zur Massenkultur hochgezogen und die Überstände auf t-PA-Immunreaktivität durch ELISA untersucht. Positive Klone wurden dann gepoolt und 4 Platten mit jeweils 1x10⁶ Zellen wurden in Medium mit 20 nmol/l Methotrexat selektioniert.

Nach 2 Wochen erschienen Methotrexat-resistente Kolonien. Sie wurden zur Konfluenz wachsen gelassen und einem Medium mit 100 nmol/l Methotrexat ausgesetzt. Resistente Zellen wurden schrittweiser Erhöhung der Methotrexat-Konzentration (300 nmol/l, 500 nmol/l, 1 μmol/l und 5 μmol/l) ausgesetzt. Die Klone wurden schließlich durch limitierte Verdünnung isoliert und die besten Produzenten des t-PA-Muteins durch Untersuchung der Überstände auf t-PA-Immunreaktivität durch ELISA ermittelt.

**Beispiel 5**

Bestimmung von t-PA, FFK2L und FK2K2L durch ELISA (enzyme linked immunosorbent assay).

Mikrotiterplatten (Firma Nunc) mit 96 Vertiefungen wurden mit Anti-t-PA-IgG (8 μg/ml) in 50 mmol/l Kaliumphosphatpuffer, pH 7,5 beschichtet und dreimal mit PBS (0,15 mol/l NaCl, 10 mmol/l Natriumphosphat, pH 7,5), 0,5 % RSA (Rinderserumalbumin) gewaschen. Die Vertiefungen wurden mit 200 μl Gewebekulturüberstand 12 Stunden bei 4° C inkubiert. Die Überstände wurden sorgfältig entfernt und die Vertiefungen dreimal mit PBS (phosphate buffered saline) gewaschen. Die Vertiefungen wurden dann mit Peroxidase-konjugiertem Ziege-Anti-t-PA-IgG, Wasserstoffperoxid und ABTS® [2,2'-Azino-di-(3-ethylbenzthiazolin-sulfonat(6))] inkubiert. Die Absorption wurde bei 405 nm gemessen und die Auswertung durch Vergleich der Absorption mit einer Eichkurve durchgeführt.

**Beispiel 6**

Bestimmung der Fibrinogen-stimulierten plasminogenolytischen Aktivität von t-PA, FFK2L und FK2K2L

Plasminogenaktivator-Aktivität wurde bestimmt durch einen indirekten spektrophotometrischen Test (Verheijen et al., Thromb. Haemostasis 48 (1982), 266-269). t-PA führt Plasminogen in die aktive Serinprotease Plasmin über, welche eine peptidische Bindung in einem chromogenen Substrat hydrolysiert, dessen Absorption bei 405 nm über einen Zeitraum von bis zu 3 Stunden verfolgt wurde. Als chromogenes Substrat wurde Tosyl-glycyl-prolyl-lysin-4-nitranilid-acetat (Chromozym® PL) verwendet. Die Tests wurden bei 25°C in 0,1 mol/l Tris-HCl, pH 7,5, 0,15 mol/l Tween 80, enthaltend 0,13 µmol/l Plasminogen, CNBr-verdautes Fibrinogen (120 µg/ml) und 0,30 mmol/l Chromozym® PL, durchgeführt.

**Beispiel 7**

Kulturüberstände von CHO-Zellen, welche konstitutiv t-PA oder FK2K2L sezernieren, wurden bezüglich der Fibrinogen-stimulierten proteolytischen Aktivität, wie in Beispiel 6 beschrieben, untersucht, gleichzeitig wurde die Menge an t-PA oder FK2K2L in den Überständen durch ELISA bestimmt. Dabei zeigte sich, daß die proteolytische Aktivität von FK2K2L durch CNBr-Fragmente von Fibrinogen etwa gleich stark (ca. 20- bis 30fach) stimuliert wurde wie die von t-PA. Es zeigte sich überraschenderweise, daß FK2K2L im Vergleich zu t-PA eine um den Faktor 3 gesteigerte spezifische Aktivität aufweist. CHO-Zellen, welche konstitutiv FK2K2L sezernieren, lösten sich schon nach dem ersten Amplifikations-Schritt von der Petri-Schale ab. Dies ist auf die Aktivierung von im Serum vorhandenem Plasminogen zur aktiven Serin-Protease Plasmin durch FK2K2L zurückzuführen. In Anwesenheit des Protease-Inhibitors Aprotinin in den Kulturüberständen wurde vorwiegend einkettiges FK2K2L, bei dessen Abwesenheit wurde ausschließlich zweikettiges FK2K2L nach gelelektrophoretischer Auftrennung immunadsorbierter Kultur-Überständen und deren Visualisierung mit Peroxidase-markiertem Antikörper gegen menschliches t-PA nachgewiesen.

Überstände von CHO-Zellen, die mit dem amplifizierbaren Expressionsvektor pSV-FFK2L-dhfr transfiziert wurden, erhöhten die proteolytische Aktivität von t-PA signifikant in Anwesenheit von CNBr-Fragmenten von Fibrinogen. Dieses Derivat weist etwa dieselbe spezifische Aktivität wie t-PA auf.

**Ansprüche**

1. Gewebsplasminogen-Aktivator (t-PA)-Mutein, **dadurch gekennzeichnet,** daß es die natürliche Aminosäuresequenz mindestens einer der Domänen Fibrinfinger und Kringel II in vervielfachter Form aufweist.

2. Gewebsplasminogen-Aktivator (t-PA)-Mutein nach Anspruch 1, **dadurch gekennzeichnet,** daß zusätzlich die Aminosäuresequenz der Domäne EGF oder/und Kringel I deletiert ist.

3. Gewebsplasminogen-Aktivator (t-PA)-Mutein nach einem der Ansprüche 1 oder 2, **dadurch gekennzeichnet,** daß er in Eukaryonten exprimiert wurde und glykosyliert ist.

4. Verfahren zur Herstellung eines t-PA-Muteins, das die natürliche Aminosäuresequenz mindestens einer der Domänen Fibrinfinger und Kringel II in vervielfachter Form aufweist, **dadurch gekennzeichnet,** daß man mehrere komplette t-PA-Gene direkt hintereinander in einen geeigneten Vektor insertiert, durch Oligonukleotid gerichtete Mutagenese diejenigen Sequenzen entfernt, die für Aminosäuren kodieren, die im Produkt nicht mehrfach vorliegen sollen, den so erhaltenen mutierten Vektor in eine geeignete Zelle einführt und amplifiziert, diejenigen Vektoren gewinnt, in welchen die Deletion stattgefunden hat und den Vektor in einer geeigneten Wirtszelle exprimiert.

5. Verfahren nach Anspruch 4 zur Herstellung eines t-PA-Muteins nach einem der Ansprüche 2 oder 3, **dadurch gekennzeichnet,** daß man vor der Insertion der t-PA-Gene in einen Vektor die für die EGF- und/oder Kringel I-Domäne kodierenden Sequenzen ebenfalls durch Oligonukleotid-gerichtete Mutagenese entfernt.

6. Verfahren nach Anspruch 4 oder 5 , **dadurch gekennzeichnet,** daß man ein Oligonukleotid mit 18 bis 26 Nukleotiden verwendet.

7. Verfahren nach einem der Ansprüche 4 bis 6 , **dadurch gekennzeichnet,** daß man zuerst durch Oligonukleotid-gerichtete Mutagenese die DNA-Sequenzen für die Aminosäuren der EGF- und Kringel I-Domäne entfernt, und dann nach der Insertion der t-PA-Gene durch Oligonukleotid-gerichtete Mutagenese die Sequenzen zwischen den beiden für die Fibrinfinderdomäne kodierenden Sequenzen, oder die Sequenzen zwischen den beiden für die Kringel II-Domäne kodierenden Sequenzen entfernt.

8. Mittel zur Gerinnselauflösung, **dadurch gekennzeichnet,** daß es ein Gewebsplasminogen-Aktivator (t-PA)-Mutein nach einem der Ansprüche 1 bis 3 enthält.

# Fig.1

## PLASMINOGEN-AKTIVATOR VOM GEWEBETYP

SCHWERE KETTE        LEICHTE KETTE

| FIBRIN-FINGER | EGF-DOMÄNE* | KRINGEL I DOMÄNE | KRINGEL II DOMÄNE | PROTEASE |

*DOMÄNE MIT HOMOLOGIE ZU EPIDERMAL GROWTH FACTOR (EGF)

Fig.2

EP 0 366 091 A2

# Fig.3

Legend:

- ﹋﹋﹋ ADENO-MAJOR-LATE PROMOTER
- ●●●● dhfr = DIHYDROFOLAT-REDUKTASE
- ▥▥▥ SV40
- ▬▬ KANINCHEN-β-GLOBIN
- ▦▦▦ t-PA
- IVS  INTERVENING SEQUENCE VON KANINCHEN-β-GLOBIN